# EUROPEAN PATENT APPLICATION

(11) **EP 4 800 715 A1**
(43) Date of publication of application: **02.09.2026**
(21) Application number: 25161135.6
(22) Date of filing: 28.02.2025
(51) Int. Cl.: G21F 3/02, G21F 3/025, G21F 3/03

(54) **RADIATION-PROTECTIVE CLINICAL UNIFORM**

(71) Applicant: Texray AB, 412 92 Göteborg (SE)
(72) Inventor: APELL, Petra, 439 37 ONSALA (SE); GELLERSTEDT, Fredrik, 413 09 GÖTEBORG (SE)
(74) Representative: Öberg & Sjöberg AB

(57) **Abstract**

A radiation-protective clinical uniform comprising: a garment for a wearer, the garment comprising: a main portion for covering at least one segment of the trunk of the wearer, and a pair of sleeves and/or a pair of trouser legs attached to the main portion for covering at least a part of two limbs of the wearer, wherein: at least a part of a front of the main portion has a first level of radiation protection which is 0.05 mm to 0.50 mm Pb eq., any remainder of the front of the main portion has a second level of radiation protection which is 0 mm to 0.20 mm Pb eq., and which is the same or lower than the first level of radiation protection, and the remainder of the front of the main portion is made from a fabric. A radiation-protective system and a use is also disclosed.

## Description

### Technical field

The technology proposed herein relates to a radiation-protective clinical uniform, and particularly to a radiation-protective clinical uniform comprising a garment for a wearer.

### Background

Ionizing radiation, such as X-ray, is commonly used in radiologically guided interventions, such as Cardiology, or Interventional Radiology (IR). During the procedure, the patient is often placed on an operating table. A source of radiation can be positioned in various angular projections in relation to the patient, such as e.g. below the operating table.

The clinical staff is exposed to the radiation during the procedures. The radiation can be direct from the primary source or scattered as secondary radiation. In particular, radiation incident on a patient or a part of a patient will scatter, both at the point where the radiation impinges on the patient but also throughout the body of the patient. Further scattering occurs as the radiation impinges on the operating table.

Current standards and practices are based on the premise that any radiation dose may result in unfavorable health effects and the development of radiation-induced diseases including glioblastoma. Reducing the radiation exposure of the clinical staff is therefore important.

Today, the clinical staff are equipped with Personal Protection Equipment (PPE) to protect them from the potentially harmful radiation. The PPE is vital as the clinical staff must be stationed next to the patient during the procedure, thus exposing themselves to scattered radiation, secondary radiation that is emitted from reflecting objects in the area around the source of radiation, such as the patient, operating table, surrounding equipment etc.

PPE for radiation protection primarily include lead garments, lead glasses, and thyroid collars. Lead garments are available in several styles, including frontal apron, wrap-around apron, and vest-skirt combinations. Frontal aprons are suitable for protecting against radiation from a front direction, whereas wrap-around aprons also provide protection for the sides and back. Vest-skirt combinations comprise a vest and a separate skirt and distribute the weight between the upper and lower body and may be provide protection from a front direction or from the front direction as well as from the sides and back.

The level of radiation protection of PPE is graded as mm Lead equivalence (mm Pb eq.). The typical standard levels are 0.25 mm, 0.35 mm and 0.50 mm Pb eq. These protective products are however very heavy, which leads to an increased health risk, such as orthopedic injuries, neck and back pain, to its user.

Attempts to reduce the weight of PPE for radiation protection mainly comprise using alternatives to lead, such as "no-leads", having combinations of e.g. antimony, wolfram, bismuth or barium, and limiting the coverage of the PPE, e.g. using a front apron instead of a wrap-around or vest-skirt combination when possible. Further, the duration of use of PPE for radiation protection is typically limited to the time that the PPE is absolutely needed.

Despite such attempts there is still a need to further reduce the weight of PPE for radiation protection during radiologically guided interventions.

### Object of the proposed technology

The proposed technology aims at providing a lighter alternative to conventional PPE for radiation protection.

It is a further object of the proposed technology to provide an alternative to conventional PPE for radiation protection that is easier and more user friendly to use.

### Summary

A first aspect of the proposed technology concerns a radiation-protective clinical uniform comprising:
a garment for a wearer, the garment comprising:
   a main portion for covering at least one segment of the trunk of the wearer, and
   a pair of sleeves and/or a pair of trouser legs attached to the main portion for covering at least a part of two limbs of the wearer,
wherein:
   at least a part of a front of the main portion has a first level of radiation protection which is 0.05 mm to 0.50 mm Pb eq.,
   any remainder of the front of the main portion has a second level of radiation protection which is 0 mm to 0.20 mm Pb eq., and which is the same or lower than the first level of radiation protection, and the remainder of the front of the main portion is made from a fabric.

The technology proposed herein accordingly involves foregoing conventional PPE for radiation protection altogether and instead integrating radiation protection into the ubiquitously used clinical uniform, such as the scrub suit, "scrubs", or the white laboratory coat "doctor's coat" that healthcare professionals wear during work in a healthcare setting.

The proposed technology is further based on the recognition that, if sufficient radiation protection is provided on or around the patient, such as with lead screens, radiation protective table skirts/curtain or a source blocking radiation protection system, e.g. from manufacturers such as ZeroGravity, Rampart ic, Egg medical, or Texray, the level of radiation-protection required for the clinical staff, i.e. the wearer of the radiation-protective clinical uniform, can be reduced such that at least part of the front of the garment may have a protection level in the range of 0.05 to 0.50 mm and the remainder being the same and lower at 0 mm to 0.20 mm Pb eq. while still providing a more than adequate protection. This lower level of radiation protection results in a significantly lower weight than conventional PPE for radiation protection. Here it was surprisingly found that a clinical uniform having a weight of as low as 1.9 kg and still providing a level of radiation protection of 0.05 mm Pb eq. could be achieved as shown in Example 1. Expressed differently, the advent of radiation-protective drapes or coverings placed on the patient has here revealed the unexpected opportunity to significantly decrease the PPE radiation protection worn by the clinical staff, which in turn has made it possible to significantly reduce the weight and produce a radiation-protective clinical uniform sufficiently light and comfortable to be worn as a scrub suit or laboratory coat throughout the working day.

Sufficient radiation protection provided on or around the patient may for example be obtained using radiation-protective coverings or drapes positioned on the patient, radiation-protective shields positioned or attached to the operating table, and/or a transparent lead screen.

By using a fabric for the remainder of the front of the main portion, the flexibility is increased, and the stiffness is decreased, for the radiation-protective clinical uniform.

As a further advantage of the proposed technology, the clinical staff, i.e. the wearer of the radiation-protective clinical uniform, will always have a basic level of protection against radiation since the radiation-protective clinical uniform may be worn as a scrub suit, i.e. surgical scrubs or nursing scrubs, generally known simply as "scrubs", or for example as a laboratory coat "doctor's coat". Thus, even if further radiation-protective shields or systems should fail, e.g. due to negligence, malfunction or if there is no time to add them during an emergency, the clinical staff has a basic protection against radiation that is significantly beyond the minimal protection afforded by conventional surgical or nursing scrubs or laboratory coats. Additionally, there is no risk that the clinical staff forgets to use the radiation protection since it is integrated in the scrubs or laboratory coat that the clinical staff is always required to wear.

As an added advantage of the proposed technology, less or no additional radiation protection needs to be donned by the clinical staff before performing or attending a radiologically guided intervention or procedure. This saves time for the clinical staff to the benefit for the patient. It saves time by making it easier and faster to commence, pause and resume, and finish the procedure. For example, it significantly simplifies situations where the clinical staff performing a procedure may require the help on consultation of a colleague, who, by wearing the radiation-protective clinical uniform, may easier enter the procedure room and/or approach the patient without lengthy procedures of donning conventional PPE for radiation protection.

Yet a further advantage of the proposed technology is that, if the situation requires additional radiation protection, then such additional radiation protection, for example in the form of conventional PPE for radiation protection, can be made lighter because a basic level of protection against radiation is already provided by the radiation-protective clinical uniform.

Specifically, if the radiation-protective clinical uniform is combined with conventional PPE for radiation protection, then the resulting combination moves more easily with the movements of the user due to the reduced moment of inertia obtained by positioning part of the radiation protective material closer to the body of the user. Further, less radiation-protective material in the conventional PPE may be needed, thus reducing the added weight of radiation protective material when entering the procedure room and donning the conventional PPE.

Further, by using a fabric for the remainder of the front of the main portion, the garment may be made more tight-fitting, which reduces the amount of radiation-protective material needed, compared to conventional PPE for radiation protection which typically is made from materials that are sensitive to folding and pinholes which makes them prone to crack and hence are difficult or impossible to sew. A tighter fit further provides for spreading the weight of the radiation-protective clinical uniform over a larger surface of the body of the wearer. This is different from a conventional lead apron which concentrates the load to the neck or shoulders of the wearer, as well being different from a conventional lead skirt concentrating the load to the hipline of the wearer.

It is understood that the radiation-protective clinical uniform provides radiation protection regardless of the presence or absence of further radiation protections such as conventional PPE for radiation protection, radiation protective coverings, and radiation protective shields. In other words, the radiation-protective clinical uniform may be used without any further or additional such radiation protection. This may be the case for clinical procedures where the expected dose of radiation to the wearer is low. Further, for such procedures, there may not be any need of any further radiation protection such as coverings or shields.

The radiation-protective clinical uniform as well as the garment, is suitable for being worn by a wearer. Expressed differently, the radiation-protective clinical uniform as well as the garment has an intended position worn by a wearer. The wearer is preferably a person attending an operating room, hybrid operating room or catheterization lab, such as a healthcare professional. The healthcare professional may be a doctor, technician or a nurse. In particular, the healthcare professional may be trained or competent in performing or assisting medical procedures such as radiologically guided interventions such as interventional radiology or cardiology procedures. It is further contemplated that the wearer could be any person in a clinical or healthcare setting or environment having a risk of being exposed to radiation. The wearer could thus further be e.g. a dentist, a receptionist, or a janitor. Generally, any person wearing a clinical uniform such as a scrub suit or doctor's coat may wear the radiation-protective clinical uniform instead to reduce their exposure to radiation.

The radiation-protective clinical uniform may alternatively be considered a radiation-protective medical uniform.

The radiation-protective clinical uniform may be worn in contact with at least part of the wearer's skin.

The clinical uniform is radiation-protective. Expressed differently, the clinical uniform protects against, or attenuates, ionizing radiation. Expressed differently, the clinical uniform may be an X-ray protective clinical uniform. The clinical uniform may comprise a radiation attenuating material, in particular an X-ray attenuating material. The radiation may be from a primary source or scattered as secondary radiation. The radiation-protective clinical uniform may be arranged or configured to attenuate X-ray radiation from a radiation source having a tube voltage of at least 40 kV, preferably at least 60 kV, and at the most 150 kV, preferably at the most 110 kV. For example, the radiation-protective clinical uniform may be arranged or configured to attenuate X-ray radiation from a radiation source having a tube voltage in the range of 40-150 kV, such as 60-110 kV.

The radiation-protective clinical uniform comprises a garment. The garment is for a wearer. Expressed differently, the garment is suitable to be worn by a wearer.

The garment comprises a main portion. The main portion of the garment is for covering least one segment of the trunk of the wearer.

The main portion may resemble or be a body of a shirt or a bodice of a dress, from which the pair of sleeves extend, when the garment is an upper body garment as described below.

Alternatively, the main portion may resemble or be a pair of briefs from which the pair of trouser legs extend, when the garment is a lower body garment as described below.

To cover a segment of the trunk the main portion should cover at least 90%, preferably all, of the area of the segment.

The trunk, or torso, is the central part or the core of the body of the wearer from which the head, neck, and limbs extend. It is understood that the trunk includes both the upper torso and the pelvic region of the wearer.

A segment of the trunk is a part of the trunk delimited by two spaced apart transverse planes. It is specified that the main portion is for covering at least one segment of the trunk of the wearer. Expressed differently, the main portion extends around the trunk of the wearer, including the front of the trunk, the sides of the trunk, and the back of the trunk. The main portion together with the two spaced apart transverse planes enclose the segment.

The segments of the trunk of the wearer are selected from the list consisting of, in order from the upper part of the trunk to the lower part, the thoracic segment, the abdominal segment, the pelvic segment, and the perineal segment. Preferably the main portion for covering at least one of the thoracic, abdominal, and pelvic segments.

The thoracic segment, or the thorax or chest, is the segment of the trunk between the neck and abdomen. The thoracic segment is also known as the upper trunk or upper torso, where the arms of the wearer extend. The thoracis segment contains the heart, lungs and thymus gland of the wearer.

The abdominal segment, or the abdomen, is the part of the trunk between the thorax, or thoracic segment, and the pelvis or pelvic segment. The abdominal segment contains the liver, spleen, stomach, gall bladder, colon, and small intestine.

The pelvic segment, or the pelvis, is the segment of the trunk between the abdomen, or abdominal segment, and the perineal segment. The pelvic segment is also known as the lower part of the trunk or torso. The pelvic segment contains the bladder and may, depending on the sex of the wearer, further contain the uterus, fallopian tubes, and ovaries.

The abdominal segment and the pelvic segment may collectively be referred to as the midsection.

The perineal segment, or the perineum, is the space between the anus and the genitals. The perineal segment may optionally be considered encompassed by the pelvic segment.

Preferably the main portion of the garment is for covering at least two or three adjacent segments of the trunk of the wearer, the segments being selected from the list consisting of the thoracic segment, the abdominal segment, the pelvic segment, and the perineal segment.

Expressed differently, the main portion may be for covering the thoracic segment and the abdominal segment, and optionally the pelvic segment, the abdominal segment and the pelvic segment, and optionally the perineal segment, or the pelvic segment and the perineal segment.

Alternatively, the main portion of the garment may be for covering all four segments of the trunk. Expressed differently, the main portion of the garment may be for covering the thoracic, abdominal, pelvic and perineal segments of the trunk.

Generally, a garment having a main portion covering at least the thoracic segment and the abdominal segment may be considered an upper body garment. An upper body garment comprises a main portion for covering at least the thoracic and abdominal segments of the trunk of the wearer and a pair of sleeves for covering at least part of the wearer's arms.

Expressed differently, the main portion of the upper body garment is for covering the upper part of the body of the wearer, e.g. between neck and waist of the wearer's body. The main portion thus is for covering the front of the upper part of the wearer's body as well as the sides and back of the upper part of the wearer's body. The main portion may thus comprise a neck opening through which the wearer's neck and/or head may protrude. The neck opening may be circular, i.e. a crew neck, V-shaped, tubular, i.e. a turtleneck, elliptical, i.e. a boat neck, or square. The main portion may comprise a hem or waist opening through which the lower part of the wearer's body may protrude. The main portion may comprise a back for covering the wearer's back. The main portion may comprise a front, or two front portions, for covering the front of the upper part of the wearer's body. Generally, for an upper body garment, the main portion may correspond to, or resembles, the body of a shirt, or the bodice of dress.

When the main portion comprises two front portions, then the two front portions may be attachable to each other. The two front portions may thus be attachable to each other by any of a zipper, buttons and buttonholes, snap fasteners, hook and loop closures, and magnetic closures. When the main portion comprises one front, or one front portion, then the front may comprise a cut extending from the neck, or upper edge, of the front and part of the way towards a hem of the upper body garment. The parts of the front separated by the cut may be attachable to each other. The parts may be attachable to each other by any of a zipper, buttons and buttonholes, snap fasteners, hook and loop closures, and magnetic closures.

The main portion may further comprise arm openings through which the arms of the wearer may extend out through the pair of sleeves.

The upper body garment may be a T-shirt, a shirt, a blouse, a sweater, a pullover, or a jersey.

An upper body garment may additionally cover the pelvic segment. Such an upper body garment may for example be a coat, dress, tunic, or smock.

The pair of sleeves of the upper body garment are preferably short sleeves so as to allow frequent and thorough washing of the under arms and hands of the wearer.

Generally, a garment having a main portion covering at least the pelvic segment and the perineal segment may be considered a lower body garment. A lower body garment comprises a main portion for covering at least the pelvic and perineal segments of the trunk of the wearer and a pair of trouser legs for covering at least part of the legs of the wearer. The lower body garment may be shorts, ¾ pants, or long-legged pants, preferably long-legged pants.

Expressed differently, the main portion of the lower body garment is for covering the lower part between waist and perineum of the wearer's body. The main portion is thus for covering the front of the lower part of the wearer's body, including the groin, as well as the sides and the rear of the lower part of the wearer's body, including the buttocks. The main portion of the lower body garment may thus correspond to or resemble a pair of briefs from which the pair of trouser legs extend.

The main portion may thus comprise a hem or waist opening through which the upper part of the wearer's body protrudes, as well as leg openings through which the legs of the wearer extends out through the pair of trouser legs. The main portion may comprise a back portion for covering the back of the lower part of the wearer's body. The main portion may comprise a front for covering the front of the wearer's body. A cut may be provided extending from the hem or waist opening of the front and part of the way towards the leg openings, e.g. forming a fly. The parts of the front separated by the cut may be attachable to each other. The parts may be attachable to each other by any of a zipper, buttons and buttonholes, snap fasteners, hook and loop closures, and magnetic closures.

A lower body garment may additionally cover the abdominal segment. Such a lower body garment may for example be an overall or a bib-and-brace overall.

The pair of trouser legs of the lower body garment preferably extend to an ankle of the wearer. This is advantageous in that it provides a larger coverage of protection compared to a conventional PPE for radiation protection. This is in particular in contrast to a conventional PPE radiation-protective apron which generally extend only to a knee area of the wearer.

Generally, a garment having a portion for covering all four segments of the trunk may be considered a whole-body garment. A whole-body garment comprises a main portion for covering the thoracic, abdominal, pelvic and perineal segments of the trunk, as well as a pair of sleeves for covering at least part of the arms of the wearer and a pair of trouser legs for covering at least part of the legs of the wearer. The whole-body garment may be a boilersuit, a coverall, or a jumpsuit.

Generally, a garment covering the abdominal segment and the pelvic segment may be considered a middle body garment. A middle body garment comprises a main portion for covering the abdominal and pelvic segments of the trunk, as well as a pair of trouser legs for covering at least part of the legs of the wearer. The middle body garment may be or comprise, a girdle, a strapless dress, or a tube dress, each together with a pair of trouser legs in the form of chaps, e.g. separate trouser legs individually attached to the main portion but where the perineal segment is not covered.

It is specified that the garment comprises a pair of sleeves and/or a pair of trouser legs attached to the main portion for covering at least a part of two limbs of the wearer.

It is understood that the pair of sleeves are attached to the main portion. It is further understood that the pair of sleeves are for covering at least a part of the wearer's arms, in particular a part of the wearer's arms extending between the shoulder and arm pit of the wearer to a position spaced apart therefrom along the wearer's arm. The pair of sleeves may thus be short sleeves, i.e. covering the wearer's arms to a position between shoulder and elbow of the wearer's arms. Alternatively, the pair of sleeves may be long sleeves, i.e. covering the wearer's arms to a position between wrist and elbow, preferably to a position at the wrists of the wearer.

It is understood that the pair of trouser legs are attached to the main portion. It is further understood that the pair trouser legs are for covering at least a part of the wearer's legs, in particular a part of the wearer's legs extending between the groin of the wearer to a position spaced apart therefrom along the wearer's leg.

The pair of trouser legs may thus be short trouser legs, i.e. covering the wearer's legs from the groin to a position between groin and knee of the wearer's legs.

Alternatively, the pair of trouser legs may be ¾ trouser legs, i.e. covering the wearer's legs from the groin to a position between knee and ankle of the wearer's legs.

Preferably, the trouser legs are long legs, i.e. covering the wearer's legs from the groin to a position between at the ankle of the wearer's legs.

It is specified that at least a part of the front of the main portion has a first level of radiation protection which is 0.05 mm to 0.50 mm Pb eq.

Expressed differently, a part, or the whole of, the front of the main portion has a radiation protection of 0.05 mm to 0.50 mm Pb eq.

The part is preferably at least 10%, more preferably at least 15%, most preferably at least 20%, such as at least 25% of the area of the front of the main portion. Preferably the part is at the most 50%, more preferably at the most 40%, most preferably at the most 35%, such as at the most 30% of the area of the front of the main portion. The part may be 10-90%, preferably 15-75%, more preferably 20-50% of the area of the front of the main portion.

The part is preferably arranged so as to cover or protect a radiation sensitive organ in the trunk of the wearer. Radiation sensitive organs include hematopoietic organs such as the bone marrow, spleen, liver, and thymus, the gastrointestinal tract, reproductive organs such as testes and ovaries, and organs such as mammary glands, lungs, kidneys, liver and thyroid gland.

The part may accordingly be arranged to generally cover the trunk of the wearer (e.g. to protect the bone marrow), to cover the thoracic segment (e.g. to protect the mammary glands, lungs and partially the thyroid gland), to cover the abdominal segment (e.g. to protect the gastrointestinal tract, kidneys and liver), and/or to cover the pelvic and perineal segments (to protect the reproductive organs).

The part may for example comprise any of the whole of the front, an upper half of the front, a lower half of the front, a center portion of the front, and a side portion of the front.

The front of the main portion is the part of the main portion facing forwards when the garment is worn by the wearer. Thus, the front of the main portion faces upwards when the garment is spread out flat on a flat, preferably horizontal, surface with the back towards the surface. The front of the main portion is further the part of the main portion covering the segment of the trunk provided on the front of the wearer. Generally, a front of the main portion may have a lower neckline where the garment is an upper body garment, and a fly or drawstring where the garment is a lower body garment.

The part comprises, or is made of, a radiation-protective material. The radiation-protective material is configured to provide the first level of radiation protection. The radiation-protective material preferably comprises a carrier substance and a radiopaque, or radiation attenuating, substance. The carrier substance and the radiopaque substance preferably form a solid composite material with the radiopaque substance distributed in, or coated onto, the carrier substance. Preferably the radiopaque substance is evenly distributed in, or evenly coated onto, the carrier substance.

The carrier substance, in the radiation-protective material, may comprise at least one polymer and the radiopaque substance may comprise at least one metal. For example, the polymer may be a thermoplastic polymer such as a polyvinyl chloride or a polyolefin and the metals may, as a powder, be as an element, oxide or alloy of for example Lead, Barium, Antimony, Bismuth, or Tungsten (Wolfram), and any combination thereof.

The solid composite material, or solid composition, may be formed into a sheet.

Alternatively, the solid composite material is formed into a plurality of filaments. It is understood that each filament may be a monofilament. The filaments may be collected and distributed regularly or irregularly to form a sheet or a fabric. The filaments may extend in a uniform direction or in random directions.

Preferably the radiation-protective material is a fabric, or fibrous material comprising the filaments. The fabric may be a woven fabric, a non-woven fabric, a knitted fabric, and/or a braided fabric.

In the woven fabric the filaments form the weft of the fabric. The woven fabric further has a warp. The warp is preferably not made of the filaments. The warp may be made of polyester.

In the non-woven fabric the filaments are arranged randomly to form a sheet. The filaments may be bonded together by chemical, mechanical, heat or solvent treatment. Alternatively, the filaments may be deposited onto, and bonded to, a carrier fabric.

In the knitted fabric the filaments form interlacing loops with loops of the same or other filaments. The knitted fabric may be weft knitted or warp knitted.

In the braided fabric the filaments interlace.

It is understood that the radiation-protective material may be composed of several layers. The layers may be identical of different.

Preferably the radiation-protective material is a woven fabric with the filaments forming the weft of the fabric. Preferably the radiation-protective fabric may be any of the radiation-protective materials described in WO2014163574A1.

The radiation-protective material is preferably flexible, or pliable.

The radiation-protective material is preferably formed as a sheet or web or fabric.

The radiation-protective material may have a thickness of at least 0.1 mm, preferably at least 0.2 mm, more preferably at least 0.5 mm. The radiation-protective material may have a thickness or at the most 10 mm, preferably at the most 5 mm, more preferably at the most 4 mm. The radiation-protective material may have a thickness of 0.1-10 mm, preferably 0.2-5 mm, more preferably 0.5-4 mm.

The radiation-protective material is preferably air-permeable. Expressed differently the radiation-protective material may be configured to allow air to pass through the radiation-protective material. The radiation-protective material may preferably have a mass transfer rate of at least 0.1 Liter air per dm² and minute, more preferably at least 1, at least 3, at least 5, or at least 10 later air per dm² and minute according to SS-EN ISO 9237, measured at 100 Pa using a 1 dm² circular pressure head.

The part may consist of the radiation-protective material. Alternatively, the part my comprise the radiation-protective material and an inner and/or outer liner. The liner may be made of the same fabric as the remainder of the front of the main portion or may be made of the same fabric as the back of the main portion described below.

The first level of radiation protection may be considered a higher level of radiation protection.

The first level of radiation protection is 0.05 mm to 0.50 mm Pb eq. Expressed differently the radiation-protective material has the first level of radiation protection.

The radiation protection in mm Pb is to be measured according to Inverse Broad Beam Geometry as described in IEC 61331-1:2014. The radiation protection in mm Pb eq. may preferably be considered minimum values at any tube voltage in the range 40-150 kV, such as 60-110 kV.

It is specified that any remainder of the front of the main portion has a second level of radiation protection which is 0 mm to 0.20 mm Pb eq., and which is the same or lower than the first level of radiation protection.

Expressed differently, if the part of the front of the main portion makes up less than all of the front, then the area not made up by the part is the remainder of the front.

The remainder is preferably at the most 90%, more preferably at the most 85%, most preferably at the most 80%, such as at the most 75% of the area of the front of the main portion. Preferably the remainder is at least 50%, more preferably at least 60%, most preferably at least 65%, such as at least 70% of the area of the front of the main portion. The remainder may be 90-10%, preferably 85-25%, more preferably 80-50% of the area of the front of the main portion.

The remainder is preferably arranged so as to cover radiation insensitive, or less radiation sensitive, organs or tissues in the trunk of the wearer.

Radiation insensitive, or less radiation sensitive, organs or tissues may include e.g. muscles, and bones.

As such, the remainder may be arranged to cover the sides of the trunk, e.g. the outermost lateral layer of skin, muscles and tissue outside the ribcage and hipbones when the front of the wearer is viewed.

Further, the remainder may be arranged to cover an organ less radiation sensitive than an organ covered by the part. Thus the part may cover an organ or tissue listed earlier than an organ covered by the remainder in the following list: i) Blood-forming cells, ii) reproductive organs, iii) gastrointestinal tract, (iv) skin, v) other major organs such as mammary glands, lungs, kidneys, liver and thyroid gland, vi) blood vessels, muscles, and bones, vii) nerves.

The remainder may thus for example comprise an upper half of the front, a lower half of the front, and a side portion of the front.

The remainder comprises, or is made of, a material. The material may be a radiation-protective material. A radiation protective material has a radiation protection level of more than 0.001 mm Pb. Eq. Where the material is a radiation protective material the radiation-protective material may be a fabric as described above for the part. Specifically, the radiation-protective material is then configured to provide the second level of radiation protection. Further, the radiation-protective material is then a fabric, or fibrous material comprising the filaments. The fabric may be a woven fabric, a non-woven fabric, a knitted fabric, and/or a braided fabric as described above. Preferably the fabric is a woven fabric with the filaments forming the weft of the fabric. Preferably the fabric may be any of the radiation-protective materials described in WO2014163574A1.

The fabric is preferably flexible, or pliable.

The fabric is preferably formed as a sheet or web or fabric.

The fabric may have a thickness of at least 0.1 mm, preferably at least 0.2 mm, more preferably at least 0.5 mm. The fabric may have a thickness or at the most 10 mm, preferably at the most 5 mm, more preferably at the most 4 mm. The fabric may have a thickness of 0.1-10 mm, preferably 0.2-5 mm, more preferably 0.5-4 mm.

The fabric is preferably air-permeable. Expressed differently the fabric may be configured to allow air to pass through the fabric. The fabric may preferably have a mass transfer rate of at least 0.1 Liter air per dm² and minute, more preferably at least 1, at least 3, at least 5, or at least 10 later air per dm² and minute according to SS-EN ISO 9237, measured at 100 Pa using a 1 dm² circular pressure head.

The remainder may consist of the fabric. Alternatively, the remainder my comprise the fabric and an inner and/or outer liner. The liner may preferably be made of the same fabric as the back of the main portion described below.

Where the material, i.e. the fabric, has a radiation protection level below 0.001 mm Pb eq., the material may be considered a radiation-transparent fabric. The radiation-transparent fabric may be the same fabric as the back of the main portion described below.

The second level of radiation protection may be considered a lower level of radiation protection.

The second level of radiation protection is 0 mm to 0.20 mm Pb eq. Expressed differently the fabric has the second level of radiation protection.

The second level of radiation protection is the same or lower than the first level of radiation protection. Expressed differently the second level of radiation protection is not higher than the first level of radiation protection.

The main portion further comprises a back. The back of the main portion may have the same or lower radiation protection level as the second level of radiation protection. Preferably the back has a level of radiation protection which is less than 0.05 mm Pb eq., preferably less than 0.01 mm Pb eq., more preferably less than 0.001 mm Pb eq.

The back of the main portion may be made of any of the materials and fabrics mentioned above for the part of front of the main portion and for the remainder of the front of the main portion. Preferably however the material used for the back of the main portion is the fabric used for the remainder of the front of the main portion. More preferably, the back of the main portion is made of a fabric having a level of radiation protection which is less than 0.0001 mm Pb eq., i.e. is radiation transparent, and which is air permeable as described above. The back of the main portion may thus be made from any commercially or conventionally available fabrics made from animal-based fibres such as wool and silk, plant-based fibres such as bamboo, cotton, flax, hemp, jute, lyocell, and rayon, and synthetic fibres such as nylon, polyester, and spandex, as well as mixtures of fibres.

Preferably the garment is an upper body garment comprising the main portion for covering the thoracic and abdominal segments, and preferably also the pelvic segment, of the trunk of the wearer and the pair of sleeves for covering at least part of the arms of the wearer,
preferably wherein the upper body garment is a T-shirt, a shirt, a blouse, a sweater, a pullover, or a jersey, or alternatively, a coat, dress, tunic, or smock.

Generally, a T-shirt, a shirt, a blouse, a sweater, a pullover, and a jersey do not cover the pelvic segment of the trunk of the wearer whereas a coat, dress, tunic, or smock do.

Generally, a T-shirt, a shirt, a blouse, a sweater, a pullover, and a jersey extend from the neck of the wearer to the waist of the wearer.

Generally, a coat, dress, tunic, or smock extend from the neck of the wearer to a position between the groin and knee of the legs of the wearer. The coat is preferably a laboratory coat or doctor's coat. The pair of sleeves of the coat, tunic or smock are preferably long sleeves so as to protect the sleeves of any underlying clothing worn by the wearer.

Preferably the garment is a lower body garment comprising the main portion for covering the pelvic and perineal segments, and optionally also the abdominal segment, of the trunk of the wearer and the pair of sleeves being a pair of trouser legs for covering at least part of the legs of the wearer,
preferably wherein the lower body garment is shorts, ¾ pants, or long-legged pants, or alternatively an overall or a bib-and-brace overall.

Generally, shorts, ¾ pants, and long-legged pants do not cover the abdominal segment, or only covers part of the abdominal
segment, of the trunk of the wearer, whereas an
overall or a bib-and-brace overall typically do.

Shorts typically extend from the waist of the wearer to a position at or above the knee of the legs of the wearer.

The waist of the wearer may be considered the border between the abdominal segment and the pelvic segment of the trunk of the wearer.

¾ pants typically extend from the waist of the wearer to a position between the knees and ankles of the legs of the wearer.

Long-legged pants typically extend from the waist of the wearer to the ankles of the legs of the wearer.

An overall or a bib-and-brace overall comprise long-legged pants integrated with at least a piece of material covering the front of the abdominal segment of the trunk of the wearer.

Preferably the radiation-protective clinical uniform comprises:
an upper body garment as described above, and
a lower body garment as described above.

This is advantageous in that it provides a versatile radiation-protective clinical uniform similar to the medical scrubs, or scrub suit, that healthcare professionals typically wear. Further, the level of radiation protection of the parts of the respective fronts of the respective main portions may be easily adjusted.

For increasing the radiation protection of the pelvic segment, as may be desired for both male and female wearers,
the main portion of the upper body garment preferably covers also the pelvic segment of the trunk of the wearer. This may be obtained by using an upper body garment in the form of a a coat, dress, tunic, or smock.

For increasing the radiation protection of the abdominal segment of the trunk of the wearer, as may be desired when the wearer is a pregnant female, the main portion of the lower body garment preferably cover also the abdominal segment of the trunk of the wearer. This may be obtained by using a lower body garment in the form of an overall or a bib-and-brace overall.

For increasing the radiation protection of both the pelvic segment and the abdominal segment of the trunk of the wearer, an upper body garment in the form of a coat, dress, tunic, or smock may be combined with a lower body garment in the form of a an overall or a bib-and-brace overall.

Alternatively, the garment is a whole-body garment comprising the main portion for covering thoracic, abdominal, pelvic and perineal segments of the trunk of the wearer, a pair of sleeves for covering at least part of the arms of the wearer, and a pair of trouser legs for covering at least part of the legs of the wearer, preferably wherein the whole-body garment is a boilersuit, a coverall, or a jumpsuit.

This is advantageous in that a whole-body garment ensures the whole radiation-protective clinical uniform is used, i.e. no part, i.e. garment, of the radiation-protective clinical uniform may be forgotten. Further, the whole-body garment prevents radiation from reaching the trunk of the wearer by passing, e.g. through a gap, between an upper body garment and a lower body garment.

For a whole body garment the pair of sleeves are preferably long sleeves and the pair of trouser legs are preferably long trouser legs.

Preferably at least part of the front of the pair of sleeves and/or pair of trouser legs has a third level of radiation protection which is 0 mm to 0.10 mm Pb eq.

This is advantageous in that it helps prevent radiation from reaching the trunk of the wearer through the pair of sleeves and/or through the pair of trouser legs.

The third level of radiation protection is preferably the same or lower than the second level of radiation protection.

The part the front of the pair of sleeves and/or pair of trouser legs is preferably adjacent to the part of the front of the main portion. This provides further protection from radiation reaching the part of the trunk of the wearer covered by the part of the front of the main portion.

The part of part of the front of the pair of sleeves
and/or pair of trouser legs may be the whole of the front part of the pair of sleeves and/or pair of trouser legs.

The remainder of the pair of sleeves and/or pair of trouser legs is preferably made from the same material or fabric as the back of the main portion.

Preferably the part of the front of the main portion is arranged to provide radiation-protection for at least one of the hematopoietic organs such as the bone marrow, spleen, liver, and thymus, the gastrointestinal tract, the reproductive organs such as testes and ovaries, and organs such as mammary glands, lungs, kidneys, liver and thyroid gland, of the wearer.

This is advantageous in that it provides additional radiation protection for radiation sensitive organs and tissues of the wearer.

Preferably the first level of radiation protection is 0.10 mm to 0.50 mm Pb eq., preferably 0.15 mm to 0.50 mm Pb eq., and/or the second level of radiation protection is 0 mm to 0.15 mm Pb eq., preferably 0.05 mm to 0.15 mm Pb eq.

These levels are especially suitable for the part and the remainder of the front of the main portion.

The first level of radiation protection may thus be 0.10 mm to 0.50 mm Pb eq., preferably 0.15 mm to 0.50 mm Pb eq., more preferably 0.20 mm to 0.50 mm Pb eq. The first level of radiation protection may thus be 0.10 mm to 0.50 mm Pb eq., 0.10 mm to 0.35 mm Pb eq., such as 0.10 mm to 0.25 mm Pb eq.

Most preferably the first level of radiation protection is 0.10 to 0.25 mm Pb eq.

The second level of radiation protection is preferably 0 mm to 0.15 mm Pb eq., for example 0.05 mm to 0.10 mm Pb eq. Most preferably the second level of radiation protection is 0.05 to 0.15 mm.

Generally, a level of radiation protection of 0 mm Pb eq. comprises or corresponds to any level of radiation protection below 0.001 mm Pb eq.

Preferably the part of the front of the main portion comprises or consists of a radiation-protective fabric comprising filaments made from a carrier substance and a radiopaque, or radiation attenuating, substance.

This is advantageous in that a radiation-protective fabric is more supple, and is more permeable to air, than a radiation-protective sheet. The carrier substance and a radiopaque, or radiation attenuating, substance are described above.

Preferably the filaments of the radiation-protective fabric have diameters of at the most 0.8 mm, preferably 0.2 mm to 0.8 mm.

This is advantageous in it further increases the suppleness of the radiation-protective fabric and accordingly increases the suppleness of the radiation-protective clinical uniform.

Preferably the radiation-protective fabric is a woven fabric, preferably wherein the filaments constitute the weft of the woven fabric.

A woven fabric has the advantage that the air-permeability and level of radiation protection may be easily controlled by controlling properties of the weaving of the woven fabric, such as the number of wefts per cm and the diameter filaments.

Preferably the warp and weft of the woven fabric are configured to arrange the filaments to undulate, preferably wherein the woven fabric is a satin weave or a twill, more preferably wherein the woven fabric is a two-shaft twill, a three-shaft twill, or a four-shaft twill.

This is advantageous in that it increases the suppleness of the woven fabric. Specifically, by arranging the filaments to undulate, or curve over and under the warp threads, the stiffness of the woven fabric is decreased. A lower number of interlacing points of weft and warp, such as in satin weave and twill weave compared to plain weave, further decreases the stiffness of the woven fabric.

Preferably the remainder of the front of the main portion comprises or consists of the woven fabric the first and second levels of radiation protection are obtained by one or more of:
a difference in the number of wefts per cm in the part and in the remainder,
a difference in filament diameter in the part and the remainder, and
a difference in the number of layers of filaments in the part and in the remainder.

This is advantageous in that it provides a simple way of obtaining the first and second levels of radiation protection. It further allows for producing the front of the main portion as a single piece of woven fabric having different levels of radiation protection in the part and the remainder.

A difference in filament diameter in the part and the remainder, and a difference in the number of layers of filaments in the part and in the remainder may also be used for nonwoven fabric and knitted fabrics.

A second aspect of the technology proposed herein refers to a radiation-protective system comprising:
a radiation-protective clinical uniform according to the first aspect of the proposed technology, and
at least one of:
   a radiation-protective covering for covering at least part of a patient on an operating table, and
   a radiation-protective shield for attachment to the operating table.

The system is advantageous in that it provides a sufficient level of radiation protection for the clinical staff using less material and with better comfort for the clinical staff.

The radiation protective covering may comprise one or more of a radiation-protective covering for covering the feet of a patient lying on an operating table, a radiation-protective covering for covering the legs of the patient, and a radiation-protective covering for covering the trunk of the patient.

The radiation protective shield may comprise one or more of a radiation-protective curtain for attachment to the side of an operating table, a radiation-protective shield attachable to an operating table, and a radiation-protective lead glass screen.

A third aspect of the technology proposed herein refers to a use of the radiation-protective clinical uniform according to the first aspect of the proposed technology or the system according to the second aspect of the proposed technology for protecting a wearer from radiation.

### Brief description of the drawings

A more complete understanding of the abovementioned and other features and advantages of the technology proposed herein will be apparent from the following detailed description of preferred embodiments in conjunction with the appended drawings, wherein:
**Fig. 1a and 1b** show a first embodiment of a radiation-protective clinical uniform according to the first aspect of the technology proposed herein being worn by clinical staff.
**Fig. 2a and 2b** show second and third embodiments of the radiation-protective clinical uniform comprising a second embodiment of an upper body garment and a second embodiment of a lower body garment.
**Fig. 2c and 2d** show fourth and fifth embodiments of the radiation-protective clinical uniform comprising a third embodiment of a lower body garment and a first embodiment of a whole-body garment.
**Fig. 3a 3b, and 3c** show a plain weave, a three-shaft twill weave, and a satin weave, respectively.
**Fig. 4a, 4b and 4c** show further embodiments of the first embodiment of the radiation-protective clinical uniform shown in Fig. 1a and 1b.
**Fig. 5** shows a clinical staff wearing the first embodiment of a radiation-protective clinical uniform and attending a patient on an operating table, the patient being at least partially covered by a radiation-protective and the operating table being provided with a radiation-protective curtain.

### Detailed description

In the figures and the below description, the same reference numerals are used for the same or related features. One or more ' added to a reference number indicates that the feature so designated is a variant of the feature bearing the reference number without the '.

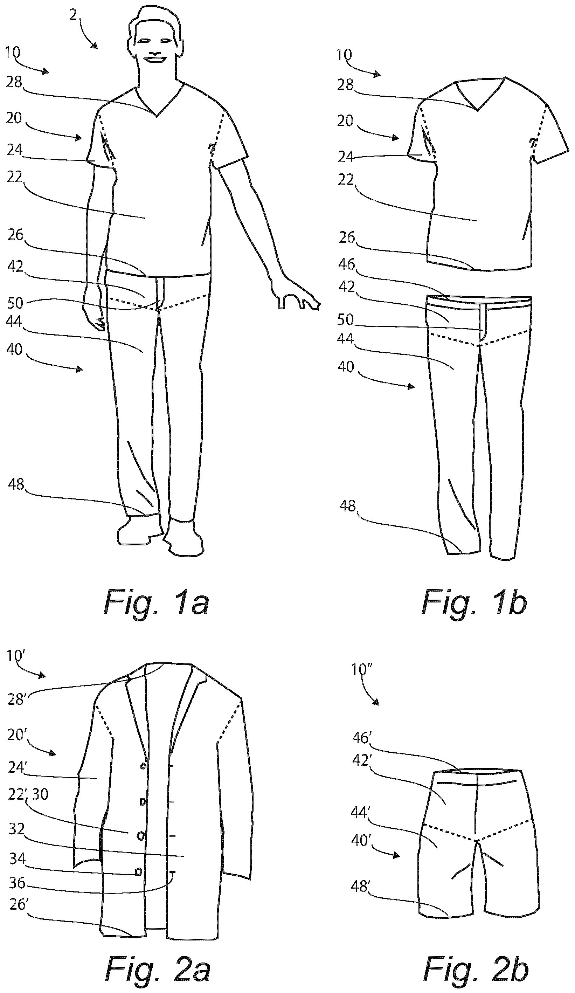

**Fig. 1a and 1b** show a first embodiment of a radiation-protective clinical uniform 10 according to the first aspect of the technology proposed herein being worn by clinical staff 2. The radiation-protective clinical uniform 10 comprises an upper body garment 20, here illustrated by a t-shirt, having a main portion 22 delimited horizontally by dashed lines at the pair of sleeves 24. The main portion 22 covers the thoracic and abdominal segments of the trunk of the clinical staff 2, or in other words covers the upper part of the trunk of the clinical staff 2 from the hem 26 at the waist of the clinical staff 2 to the neck, or neck opening, 28. The main portion 22 has a front as seen, and a back (not shown). The whole of the front of the main portion 22 has a first level of radiation protection which here is 0.05 mm Pb eq. The front of the pair of sleeves 24 here has a third level of radiation protection which also is 0.05 mm Pb eq. The front of the main portion 22 is made of a radiation-protective fabric comprising filaments comprising a carrier substance and a radiopaque substance. The radiation-protective fabric was manufactured as described in WO2014163574A1. The back of the main portion, and the back of the pair of sleeves, is made of cotton fabric.

The radiation-protective clinical uniform 10 further comprises a lower body garment 40, here illustrated by a pair of long-legged trousers, having a main portion 42 delimited vertically by dashed lines at the pair of trouser legs 44. The main portion 42 covers the pelvic and perineal segments of the trunk of the clinical staff 2, or in other words covers the lower part of the trunk of the clinical staff 2 from the pair of trouser legs 44 to the waist 46. The pair of trouser legs 44 further extend to the hem 48 of the lower body garment 40. The main portion 42 has a front as seen, and a back (not shown). A fly 50 is provided on the front of main portion 42 to simplify donning the lower body garment 40. The whole of the front of the main portion 42 has a first level of radiation protection which here is 0.05 mm Pb eq. The front of the pair of trouser legs 44 here has a third level of radiation protection which also is 0.05 mm Pb eq. The front of the main portion 42 is made of a radiation-protective fabric comprising filaments comprising a carrier substance and a radiopaque substance. The radiation-protective fabric was manufactured as described in WO2014163574A1. The back of the main portion 42, and the back of the pair of trouser legs 44, is made of cotton fabric. As seen the radiation-protective clinical uniform 10 may be worn instead of a conventional scrub suit.

**Fig. 2a and 2b** show second and third embodiments of the radiation-protective clinical uniform comprising a second embodiment of an upper body garment and a second embodiment of a lower body garment.

Fig. 2a thus shows a radiation-protective medical uniform 10' comprising an upper body garment 20' here represented by a coat, i.e. a laboratory or doctors coat having a main portion 22' delimited horizontally by dashed lines at the pair of sleeves 24', here being long sleeves. The main portion 22' covers the thoracic and abdominal segments, and also the pelvic segment, of the trunk of the clinical staff 2, or in other words covers the upper part, as well as part of the lower part, of the trunk of the clinical staff 2 from the hem 26' between the waist and knee of the clinical staff 2 to the neck, or neck opening, 28'. The main portion 22' has a front made up of two front portions 30, 32 which are attachable to each other by means of buttons 34 and buttonholes 36. The whole of the front of the main portion 22' has a first level of radiation protection which here is 0.05 mm Pb eq. The front of the sleeves here has a third level of radiation protection which also is 0.05 mm Pb eq. The front of the main portion 22' is made of a radiation-protective fabric comprising filaments comprising a carrier substance and a radiopaque substance. The radiation-protective fabric was manufactured as described in WO2014163574A1. The back of the main portion, and the back of the pair of sleeves 24', is made of cotton fabric. The upper body garment 20' may further be combined with any of the lower body garments 40, 40' and 40'', the two latter being described below.

Fig. 2b shows a radiation-protective clinical uniform 10" comprising a lower body garment 40', here illustrated by a pair of shorts, having main portion 42' delimited vertically by dashed lines at the pair of trouser legs 44'. The main portion 42' covers the pelvic and perineal segments of the trunk of the clinical staff 2, or in other words covers the lower part of the trunk of the clinical staff 2 from the pair of trouser legs 44' to the waist 46'. The pair of trouser legs 44' further extend to the hem 48' of the lower body garment 40'. The main portion 42' has a front as seen, and a back (not shown). The whole of front of the main portion 42' has a first level of radiation protection which here is 0.05 mm Pb eq. The front of the pair of trouser legs 44' here has a third level of radiation protection which also is 0.05 mm Pb eq. The front of the main portion 42' is made of a radiation-protective fabric comprising filaments comprising a carrier substance and a radiopaque substance. The radiation-protective fabric was manufactured as described in WO2014163574A1. The back of the main portion 42', and the back of the pair of trouser legs 44', is made of cotton fabric. The lower body garment 40' may be combined with any of the upper body garments 20 and 20'.

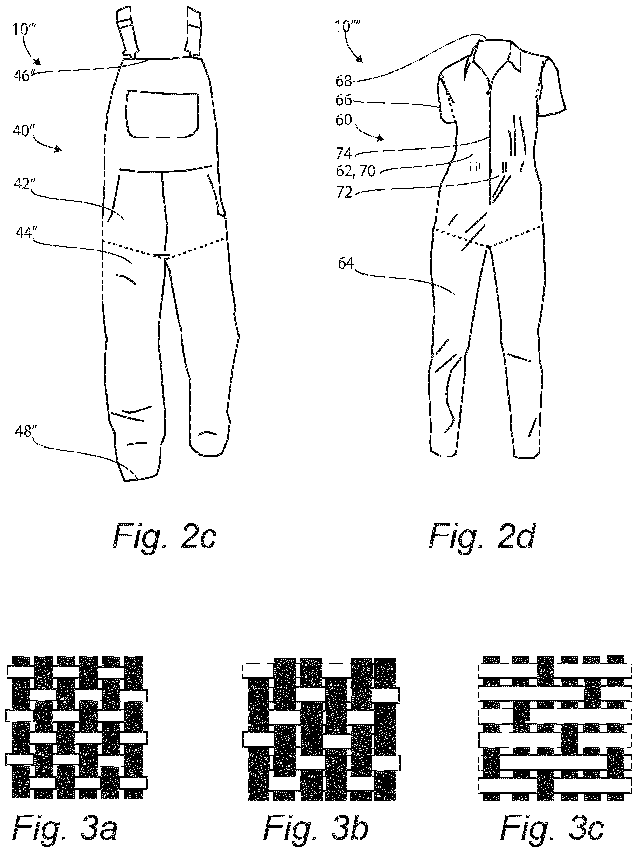

**Fig. 2c and 2d** show fourth and fifth embodiments of the radiation-protective clinical uniform comprising a third embodiment of a lower body garment and a first embodiment of a whole-body garment.

Fig. 2c thus shows radiation-protective clinical uniform 10‴ comprising a lower body garment 40", here illustrated by a bib-and-braces overall, having a main portion 42'' delimited vertically by dashed lines at the pair of trouser legs 44''. The main portion 42" covers the abdominal, pelvic and perineal segments of the trunk of the clinical staff 2, or in other words covers the lower part of the trunk and part of the upper part of the trunk of the clinical staff 2 from the pair of trouser legs 44" to the upper edge 46'' of the bib part. The pair of trouser legs 44'' further extend to the hem 48'' of the lower body garment 40". The main portion 42" has a front as seen, and a back (not shown). The whole of the front of the main portion 42" has a first level of radiation protection which here is 0.05 mm Pb eq. The front of the pair of trouser legs 44" here has a third level of radiation protection which also is 0.05 mm Pb eq. The front of the main portion 42" is made of a radiation-protective fabric comprising filaments comprising a carrier substance and a radiopaque substance. The radiation-protective fabric was manufactured as described in WO2014163574A1. The back of the main portion 42'', and the back of the pair of trouser legs 44", is made of cotton fabric.

Fig. 2d shows a radiation-protective clinical uniform 10ʺʺ comprising a whole-body garment 60 here illustrated by an overall having a main portion 62 delimited vertically by dashed lines at the pair of trouser legs 64 and horizontally by dashed lines at the pair of sleeves 66. The main portion 62 covers the thoracic, abdominal, pelvic and perineal segments of the trunk of the clinical staff 2, or in other words covers the whole of the trunk of the clinical staff 2 from the pair of trouser legs 64 to the neck 68. The main portion 62 has a front as seen, and a back (not shown). The front is partially divided into two front portion 70, 72 which may be attached to each other using zipper 74. The whole of the front of the main portion 62 has a first level of radiation protection which here is 0.05 mm Pb eq. The front of the pair of trouser legs 64 and the front of the pair of sleeves 66 here has a third level of radiation protection which also is 0.05 mm Pb eq. The front of the main portion 62 is made of a radiation-protective fabric comprising filaments comprising a carrier substance and a radiopaque substance. The radiation-protective fabric was manufactured as described in WO2014163574A1. The back of the main portion 62, and the back of the pair of trouser legs 64 and pair of sleeves 66, is made of cotton fabric.

**Fig. 3a, 3b, and 3c** show a plain weave, a three-shaft twill weave, and a satin weave, respectively of woven radiation-protective fabrics comprising filaments comprising a carrier substance and radiopaque substance. The filaments make up the weft and extend horizontally (white) and the warp threads extend vertically (black) in the weaves shown. As seen, the twill weave, e.g. the three-shaft twill weave shown in Fig. 3b, as well as the satin weave shown in Fig. 3c, have fewer interlacing points of weft and warp compared to plain weave which render fabrics made with these weaves more flexible and thus more suitable for use in the radiation-protective clinical uniform.

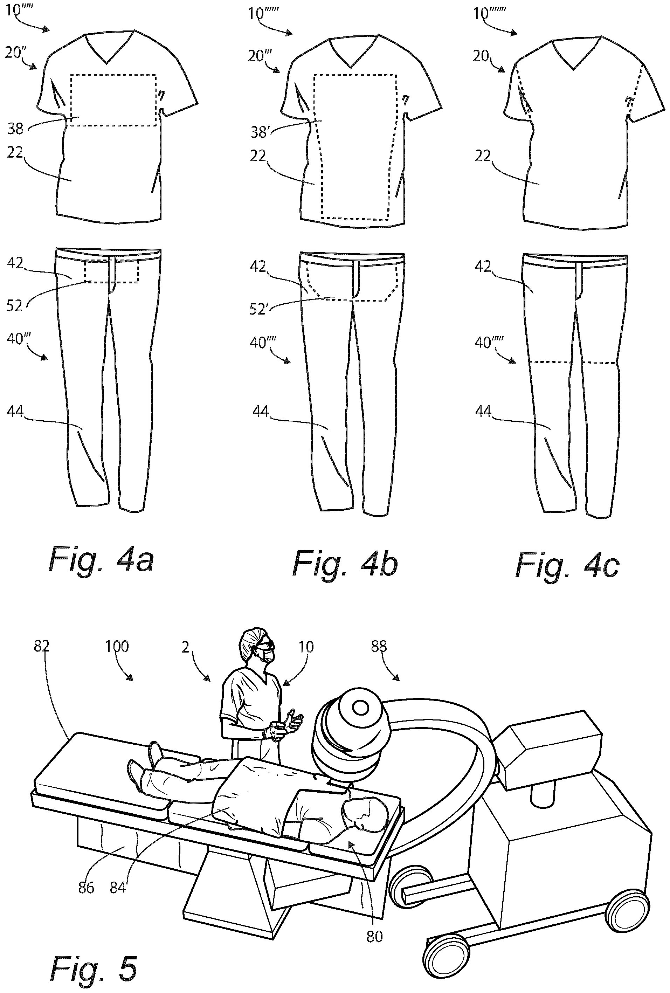

**Fig. 4a, 4b and 4c** show further embodiments of the first embodiment of the radiation-protective clinical uniform shown in Fig. 1a and 1b.

Fig. 4a thus shows an embodiment of the radiation-protective clinical uniform 10ʺ‴ wherein a part 38 of the front of the main portion 22 of the upper body garment 20'' has a first level of radiation protection, such as 0.15 mm Pb eq., whereas the remainder of the front of the main portion 22 has a second, lower, level of radiation protection such as 0 mm to 0.05 mm Pb eq. The part 38 is arranged to cover the breasts and mammary glands of the clinical staff 2. Similarly, only a part 52 of the front of the main portion 42 of the lower body garment 40''' has the first level of radiation protection, whereas the remainder of the front of the main portion 42 has the second, lower, level of radiation protection. The part 52 is arranged to cover the reproductive organs of the clinical staff 2. This significantly reduces the weight of the radiation-protective clinical uniform 10‴ʺ. The remainder of the front of the respective main portions 22, 42 may even have no radiation protection for a very low weight.

Fig. 4b shows an embodiment of the radiation-protective clinical uniform 10‴‴ wherein the part 38' of the front of the main portion 22 of the upper body garment 20, having the first level of radiation protection described directly above, has a larger extension covering the breasts and the center of the thoracic and abdominal segments providing a greater coverage of the trunk of the clinical staff 2 while not covering the less radiation sensitive lateral sides of the upper part of the trunk. As for Fig. 4a, the remainder of the front of the main portion 22 has the second, lower, level of radiation protection. Similarly, the part 52' of the front of the main portion 42 of the lower body garment 40"" having the first level of radiation protection now covers more of the pelvic segment of the trunk of the clinical staff, leaving out only the less radiation sensitive lateral sides of the lower part of the trunk. As for Fig. 4a, the remainder of the front of the main portion 42 has the second, lower, level of radiation protection. This reduces the weight of the radiation-protective clinical uniform 10‴‴ without any significant decrease in radiation protection.

Fig. 4c shows an embodiment of the radiation-protective clinical uniform 10‴‴′ wherein the upper body garment 20 is the same as in Fig. 1a and 1b, but wherein the lower body garment 40‴ʺ has a first level of radiation protection of 0.05 mm Pb eq. over the whole of the front of the main portion 42, and also over the front of the pair of trouser legs 44 down to the knees as shown by the horizontal dashed lines. This provides a good coverage while providing for some further weight reduction compared to the radiation-protective clinical uniform shown in Fig. 1a and 1b.

**Fig. 5** shows a clinical staff 2 wearing the first embodiment of the radiation-protective clinical uniform 10 and attending a patient 80 on an operating table 82, the patient being at least partially covered by a radiation-protective covering 84 and the operating table being provided with a radiation-protective curtain 86. A C-arm medical imaging machine 88 is further shown and used to image the interior structures and tissues of the patient 80. The radiation-protective clinical uniform 10 and at least one of the radiation-protective covering 84 and the radiation-protective curtain 86, which represents a radiation-protective shield, constitute a system 100. Thus, by using at least one of the radiation-protective covering 84 and the radiation-protective curtain 86 the clinical staff 2 may obtain a sufficient level of radiation protection using only the radiation-protective clinical uniform 10 and without using conventional PPE for radiation protection.

### Example 1. A prototype radiation-protective clinical uniform

A prototype radiation-protective clinical uniform was manufactured as follows.

A radiation-protective and air-permeable fabric was manufactured as described in WO2014163574A1 with the following specification. The filament diameter was 0.65 mm. Number of wefts was 21 per cm on the loom. The warp was a polyester thread of 0.2 mm diameter. The weight of the manufactured radiation-protective permeable fabric was 2.1 kg per m². The attenuating component, i.e. radiopaque substance, was Barium sulphate, and it was dispersed in a polymeric polyvinyl chloride (PVC) matrix or carrier substance. The radiation protection was 0.05 mm Pb eq. The attenuating capacity of the radiation-protective fabric was measured to be 61% at a tube voltage of 60 kV. The radiation-protective clinical uniform was manufactured in size Large and comprised a short-sleeved T-shirt and a pair of long-legged pants. The T-shirt was sewn with the front of the main portion made from the radiation-protective fabric, wrapped into a cotton compartment, i.e. inner and outer liner made from cotton fabric, 120 g/m², and the back of the main portion made from the cotton fabric. The area of the front of the T shirt was measured to be 0.46 m². The front of the sleeves was also made from the radiation protective fabric, wrapped in the cotton fabric. The back of the sleeves was made from the cotton fabric. The pants were sewn with the front of the main portion and the trouser legs down to the knees in the radiation-protective fabric and the back of the main portion, the back of the trouser legs, and the lower part of the front of the trouser legs below the knee, in the cotton fabric. The total surface of the radiation-protective fabric in the pants was 0,2 m². The combined weight of the T-shirt and pants was 1,9 kg. The radiation-protective clinical uniform was tested by a test person and found to provide only negligible discernable additional weight compared to a conventional pair of cotton pants and a conventional T-shirt together weighing 1 kg.

### Item list

- 2: clinical staff
- 10: radiation-protective clinical uniform
- 20: upper body garment
- 22: main portion
- 24: pair of sleeves
- 26: hem
- 28: neck
- 30: first front portion
- 32: second front portion
- 34: button
- 36: buttonhole
- 38: part of front of main portion
- 40: lower body garment
- 42: main portion
- 44: pair of trouser legs
- 46: waist
- 48: hem
- 50: fly
- 52: part of front of main portion
- 60: whole-body garment
- 62: main portion
- 64: pair of trouser legs
- 66: pair of sleeves
- 68: neck
- 70: first front portion
- 72: second front portion
- 74: zipper
- 80: patient
- 82: operating table
- 84: radiation-protective covering
- 86: radiation-protective curtain
- 88: C-arm medical imaging machine
- 100: system

## Claims

1. A radiation-protective clinical uniform (10) comprising:
a garment (20) for a wearer (2), the garment comprising:
a main portion (22) for covering at least one segment of the trunk of the wearer, and
a pair of sleeves (24) and/or a pair of trouser legs (44) attached to the main portion for covering at least
a part of two limbs of the wearer,
wherein:
at least a part of a front of the main portion (22) has a first level of radiation protection which is 0.05 mm to 0.50 mm Pb eq.,
any remainder of the front of the main portion has a second level of radiation protection which is 0 mm to 0.20 mm Pb eq., and which is the same or lower than the first level of radiation protection, and
the remainder of the front of the main portion is made from a fabric.

2. The radiation-protective clinical uniform (10) according to claim 1, wherein the garment is an upper body garment (20) comprising the main portion for covering the thoracic and abdominal segments, and preferably also the pelvic segment, of the trunk of the wearer (2) and the pair of sleeves (24) for covering at least part of the arms of the wearer, preferably wherein the upper body garment is a T-shirt, a shirt, a blouse, a sweater, a pullover, or a jersey, or alternatively, a coat, dress, tunic, or smock.

3. The radiation-protective clinical uniform (10) according to claim 1, wherein the garment is a lower body garment (40) comprising the main portion (42) for covering the pelvic and perineal segments, and optionally also the abdominal segment, of the trunk of the wearer (2) and the pair of sleeves being a pair of trouser legs (44) for covering at least part of the legs of the wearer,
preferably wherein the lower body garment is shorts, ¾ pants, or long-legged pants, or alternatively an overall or a bib-and-brace overall.

4. The radiation-protective clinical uniform (10) according to any preceding claim, wherein the radiation-protective clinical uniform comprises:
an upper body garment (20) according to claim 2, and
a lower body garment (40) according to claim 3.

5. The radiation-protective clinical uniform (10) according to claim 1, wherein the garment is a whole-body garment (60) comprising the main portion (62) for covering thoracic, abdominal, pelvic and perineal segments of the trunk of the wearer (2), a pair of sleeves (66) for covering at least part of the arms of the wearer, and a pair of trouser legs (64) for covering at least part of the legs of the wearer, preferably wherein the whole-body garment is a boilersuit, a coverall, or a jumpsuit.

6. The radiation-protective clinical uniform (10) according to any preceding claim, wherein at least part of the front of the pair of sleeves (24) and/or pair of trouser legs (44) has a third level of radiation protection which is 0 mm to 0.10 mm Pb eq.

7. The radiation-protective clinical uniform (10) according to any preceding claim, wherein the part of the front of the main portion (22, 42, 62) is arranged to provide radiation-protection for at least one of the hematopoietic organs such as the bone marrow, spleen, liver and thymus, the gastrointestinal tract, the reproductive organs such as testes and ovaries, and organs such as mammary glands, lungs, kidneys, liver and thyroid gland, of the wearer.

8. The radiation-protective clinical uniform (10) according to any preceding claim, wherein the first level of radiation protection is 0.10 mm to 0.50 mm Pb eq., preferably 0.15 mm to 0.50 mm Pb eq., and/or the
second level of radiation protection is 0 mm to 0.15 mm Pb eq., preferably 0.05 mm to 0.15 mm Pb eq.

9. The radiation-protective clinical uniform (10) according to any preceding claim, wherein the part of the front of the main portion (22) comprises or consists of a radiation-protective fabric comprising filaments made from a carrier substance and a radiopaque, or radiation attenuating, substance.

10. The radiation-protective clinical uniform (10) according to claim 9, wherein the filaments of the radiation-protective fabric have diameters of at the most 0.8 mm, preferably 0.2 mm to 0.8 mm.

11. The radiation protective clinical uniform (10) according to any of the claims 9-10, wherein the radiation-protective fabric is a woven fabric.

12. The radiation-protective uniform (10) according to claim 11, wherein the warp and weft of the woven fabric are configured to arrange the filaments to undulate, preferably wherein the woven fabric is a twill, more preferably wherein the woven fabric is a two-shaft twill, a three-shaft twill, or a four-shaft twill.

13. The radiation-protective clinical uniform (10) according to any of the claims 11-12, wherein the remainder of the front of the main portion (22) comprises or consists of the woven fabric according to any of the claims 11-12, and wherein the first and second levels of radiation protection are obtained by one or more of:
a difference in the number of wefts per cm in the part and in the remainder,
a difference in filament diameter in the part and the remainder, and
a difference in the number of layers of filaments in the part and in the remainder.

14. A radiation-protective system (100) comprising a radiation-protective clinical uniform (10) according to any preceding claim, and
at least one of:
a radiation-protective covering (84) for covering at least part of a patient (80) on an operating table (82), and
a radiation-protective shield (86) for attachment to an operating table.

15. Use of the radiation-protective clinical uniform (10) according to any of the claims 1-13 or the system (100) according to claim 15 for protecting a wearer (2) from radiation.
